# EUROPEAN PATENT APPLICATION

(11) **EP 2 380 495 A1**
(43) Date of publication of application: **26.10.2011**
(21) Application number: 11161446.7
(22) Date of filing: 07.04.2011
(51) Int. Cl.: A61B 6/00, G03B 42/02

(54) **Radiographic image detection device and radiographic imaging system**

(30) Priority: 22.04.2010 JP 2010098604
(71) Applicant: FUJIFILM Corporation, Tokyo (JP)
(72) Inventor: Ohta, Yasunori, Kanagawa (JP)
(74) Representative: Klunker . Schmitt-Nilson . Hirsch

(57) **Abstract**

An electronic cassette (12) includes a handle (23) suspended on a cable (17), and a cassette main body (24) suspended together with the handle (23) on the cable (17) when being mounted to the handle (23). The cassette main body (24) has a power source (52) for supplying electric power to various circuits and the like in the cassette main body (24) when the cassette main body (24) is removed from the handle (23), and an image memory (49) for storing the image data. Image-taking can be performed in a state that the cassette main body (24) is removed from the handle (23).

## Description

### FIELD OF THE INVENTION

The present invention relates to a radiographic image detection device for generating a radiographic image by detecting radioactive rays that have been transmitted through a subject and a radiographic imaging system using the radiographic image detection device, and more particularly to a radiographic image detection device provided with a handle for increasing portability and a radiographic imaging system using the radiographic image detection device provided with the handle.

### BACKGROUND OF THE INVENTION

A flat panel detector (FPD) which includes a radiation sensitive layer disposed on a thin film transistor (TFT) active matrix substrate so as to directly convert radioactive rays such as X rays into digital data has been put into practical use in recent years. Further, a portable radiographic image detection device (also referred to as "electronic cassette"), which uses the FPD to generate a radiographic image expressed by radioactive rays that have been irradiated to and transmitted through a subject, and stores the generated radiographic image, has been put into practical use. The radiographic image detection device is composed of a plurality of expensive electronic components. In fear of being broken by drop impact, the radiographic image detection device includes a handle for increasing portability provided on its side surface.

As the radiographic image detection device is provided with the handle and has a shape different from that of a film cassette defined by an ISO standard or that of a computed radiography (CR) cassette based on the ISO standard, the radiographic image detection device cannot be set to a shooting table for the film cassette or the CR cassette. Therefore, it is necessary to purchase another shooting table for the radiographic image detection device, thus leading to increase in cost burden for medical institutions.

Further, when taking an image of a patient sitting on a wheelchair, the radiographic image detection device is inserted between a backrest of the wheelchair and the patient in some cases. In such a case, there is a problem that an insertion direction of the radiographic image detection device provided with the handle is limited. This is because a width of the radiographic image detection device in a vertical orientation is different from that in a horizontal orientation due to the height of the handle provided to the radiographic image detection device, and the height of the handle makes it impossible to insert the radiographic image detection device between arm rests of the wheelchair in a state that the side of the radiographic image detection device to which the handle is mounted is vertical to a clearance between the arm rests.

According to Japanese Patent Laid-Open Publication No. 2004-347841, for example, there is known a radiographic image detection device which can be suspended on a wire so as not to be dropped and broken. Additionally, according to Japanese Patent Laid-Open Publication No. 2004-246384, for example, there is known a radiographic image detection device to which a handle is detachably mounted for the purpose of solving the problems caused by the handle.

In the case where the radiographic image detection device is suspended on the wire, the wire may get in the way of taking an image of a subject depending on a positional relationship between the subject and the wire.

### SUMMARY OF THE INVENTION

In view of the above, an object of the present invention is to provide a radiographic image detection device and a radiographic imaging system, in which suspension of the radiographic image detection device does not limit image-taking, while achieving an advantage of a detachably mounted handle and preventing the radiographic image detection device from being broken by suspending the radiographic image detection device.

In order to achieve the above and other objects, a radiographic image detection device of the present invention includes a handle suspended on a cable, and a main body having a detector for detecting radioactive rays which has been transmitted through a subject to generate a radiographic image. The main body is detachably mounted to the handle, and suspended together with the handle on the cable when being mounted to the handle.

The main body has a first power source for supplying electric power when the main body is removed from the handle, and a memory for storing the radiographic image, such that image-taking can be performed without the handle.

The handle has a communication section for establishing communication with an external device through the cable. Further, the handle has a second power source for supplying electric power to the main body when the main body is mounted to the handle. The second power source has a battery which is charged when the handle is connected to the cable, and supplies electric power to the main body when the handle is removed from the cable.

A radiographic imaging system of the present invention includes a radiation generator for irradiating a subject with radioactive rays, the radiographic image detection device described above, and a controller for controlling the radiation generator and the radiographic image detection device.

According to the present invention, provision of the detachable handle enables increase in portability of the radiographic image detection device when the main body is mounted on the handle, downsizing of the radiographic image detection device by removing the handle, and increase in usability of the radiographic image detection device for the conventional shooting tables. Further, since the handle is suspended on the cable, it is possible to prevent the radiographic image detection device from being dropped and broken. Furthermore, since the cable can be removed together with the handle from the main body, the cable does not get in the way of image-taking.

Since image-taking can be performed in a state that the main body is removed from the handle, image-taking also can be performed in a position unreachable for the cable. Further, when the main body is mounted to the handle, the second power source of the handle supplies electric power to the main body, and therefore the remaining capacity of the battery does not limit the image-taking.

### DESCRIPTION OF THE DRAWINGS

The above objects and advantages of the present invention will become easily understood by one of ordinary skill in the art when the following detailed description of the preferred embodiments would be read in connection with the accompanying drawings, wherein:
Figure 1 is a schematic view illustrating a structure of a radiographic imaging system according to an embodiment of the present invention;
Figure 2 is a perspective view illustrating an electronic cassette in a suspended state;
Figure 3 is an exploded perspective view illustrating a structure of the electronic cassette;
Figure 4 is a block diagram showing an electric structure of the electronic cassette;
Figure 5 is a block diagram showing a structure of a TFT active matrix substrate;
Figure 6 is an explanatory view illustrating the electronic cassette inserted under a lying subject;
Figure 7 is an explanatory view illustrating the electronic cassette held by the lying subject;
Figure 8 is an explanatory view illustrating a subject sitting on a wheelchair during image-taking;
Figure 9 is a perspective view illustrating a cassette main body to be set to a shooting table for a CR cassette;
Figure 10 is an explanation view illustrating the cassette main body to be inserted between arm rests of the wheelchair such that long sides of the cassette main body are aligned with a clearance between the arm rests;
Figure 11 is a flow chart showing an image data generating processing;
Figure 12 is a perspective view illustrating the cassette main body having side surfaces orthogonal to each other to each of which a handle can be mounted; and
Figure 13 is a block diagram showing an electric structure of the electronic cassette according to another embodiment of the present invention.

### DESCRIPTION OF THE PREFERRED EMBODIMENTS

Hereinafter, preferred embodiments of the present invention are described in detail. However, the present invention is not limited thereto.

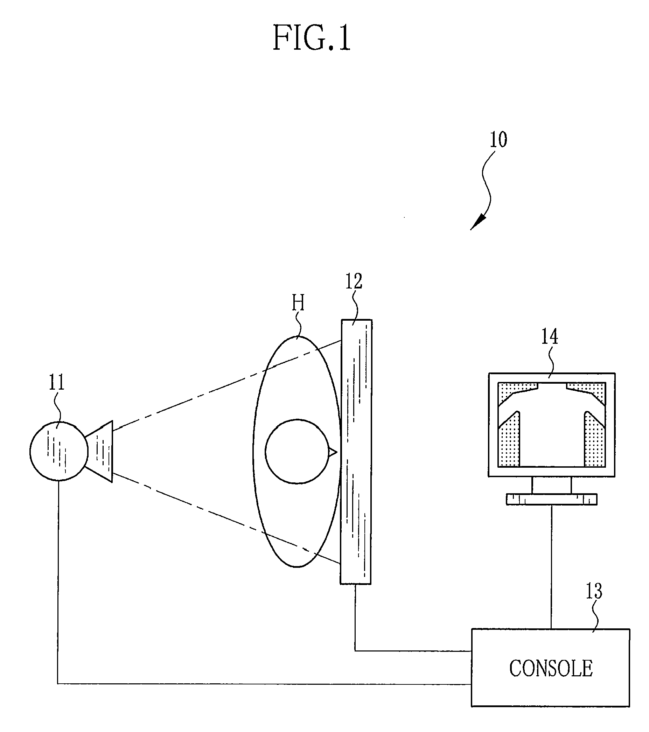

As shown in Fig 1, a radiographic imaging system of the present invention, for example, an X-ray imaging system 10 includes an X-ray generator 11, a radiographic image detection device (hereinafter referred to as electronic cassette) 12, a console 13, and a monitor 14. Under the control of the console 13, the electronic cassette 12 detects X rays that have been irradiated from the X-ray generator 11 and have been transmitted through a subject H, and generates an X-ray image. The console 13 subjects the X-ray image sent from the electronic cassette 12 to various kinds of image processing and displays the X-ray image on the monitor 14.

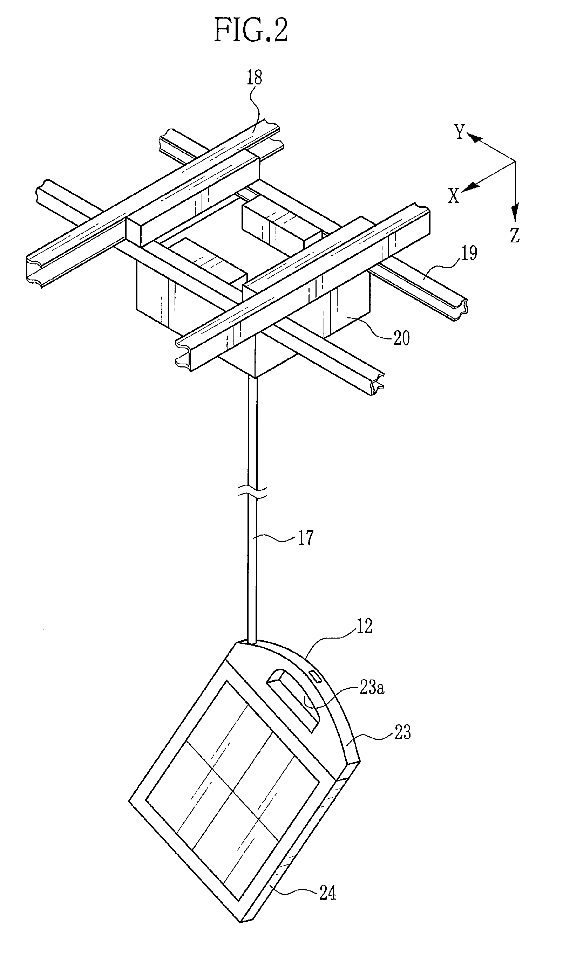

As shown in Fig. 2, the electronic cassette 12 is suspended on a cable 17 in a vertical direction Z. The cable 17 is used such that control signals and X-ray images are sent and received between the electronic cassette 12 and the console 13, and supplies electric power to the electronic cassette 12. The cable 17 has strength for holding the electronic cassette 12 in a suspended state. The cable 17 is supported by a dolly section 20. The dolly section 20 is held in a movable manner in an X direction perpendicular to the Z direction and a Y direction perpendicular to the Z direction and the X direction by a first rail 18 disposed along the X direction and a second rail 19 disposed along the Y direction. The dolly section 20 contains a well-known spring balancer (not shown) for pulling up the cable 17 at a pulling force corresponding to a weight of the electronic cassette 12 by exerting spring biasing force. The spring balancer makes it possible for the electronic cassette 12 to stop at an arbitrary height.

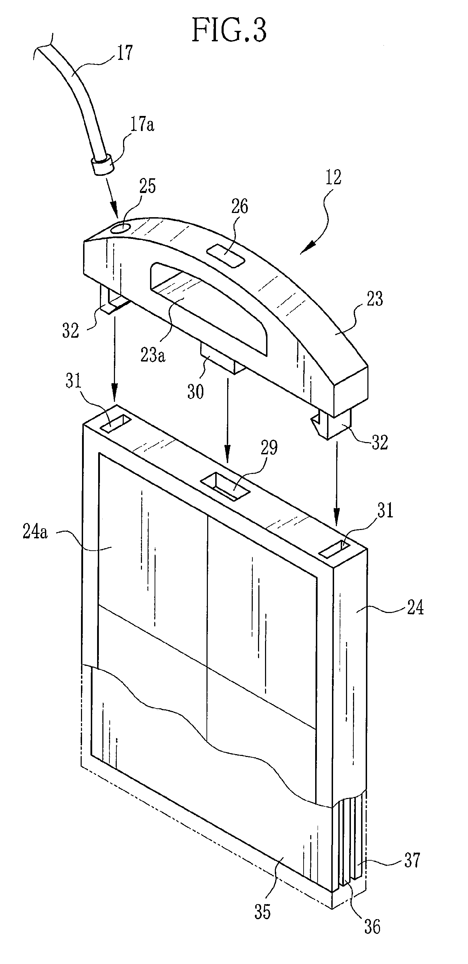

As shown in Fig. 3, the electronic cassette 12 consists of a handle 23 to which the cable 17 is detachably attached, and a cassette main body 24 which is detachably mounted to the handle 23. The cassette main body 24 is configured to perform image-taking whether it is in a state of being mounted to the handle 23 or in a state being removed from the handle 23.

A front surface of the handle 23 has an approximately circular arc shape, and includes at its central portion an opening 23a into which a hand is inserted to grip the handle 23. An upper surface of the handle 23 includes a socket 25 into which a connector 17a provided at a distal end of the cable 17 is inserted, and a connection canceling button 26 operated for separating the cassette main body 24 from the handle 23. The connector 17a has a function of engaging with the socket 25 as well as a function of canceling the engagement with the socket 25. When the connector 17a is engaged with the socket 25, the connector 17a exerts engagement strength for sustaining the weight of the electronic cassette 12.

A lower surface of the handle 23 is provided with a connector 30 and a pair of engagement claws 32. The connector 30 electrically connects the cassette main body 24 to the handle 23 upon being inserted into a socket 29 provided in an upper surface of the cassette main body 24. The pair of engagement claws 32 is inserted into and engaged with a pair of engagement holes 31 provided in the upper surface of the cassette main body 24. The pair of engagement claws 32 exerts engagement strength for sustaining the cassette main body 24 while being engaged with the engagement holes 31, and has a mechanism for canceling the engagement with the engagement holes 31 upon operation of the connection canceling button 26.

The cassette main body 24 has a detection surface 24a to which X rays are irradiated. Inside the cassette main body 24, there are disposed, in order from the detection surface 24a side, a grid 35 that absorbs scattered radiation of the X rays that arises in conjunction with the X rays being transmitted through the subject H, a X-ray detection panel 36 that detects the X rays, and a lead plate 37 that absorbs back scattered radiation of X rays.

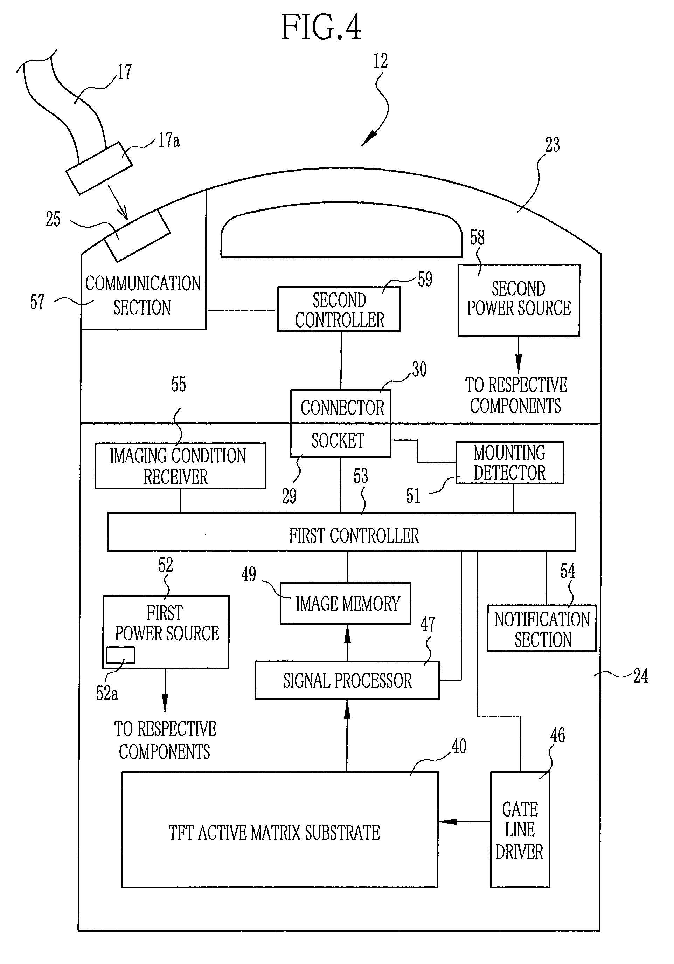

The X-ray detection panel 36 consists of the TFT active matrix substrate 40 (see Fig. 4) and a charge generation layer (not shown) laminated on the TFT active matrix substrate 40. The charge generation layer absorbs X rays and converts them into electric charges. As is well known, the charge generation layer is, for example, non-crystalline a-Se (amorphous selenium) whose main component is selenium. For example, a content percentage of the selenium in the a-Se is equal to or more than 50%. The charge generation layer converts the irradiated X rays into electric charges. Further, the charge generation layer generates, inside of itself, electric charges (electron-hole pairs) of an electric charge amount corresponding to the amount of the irradiated X rays.

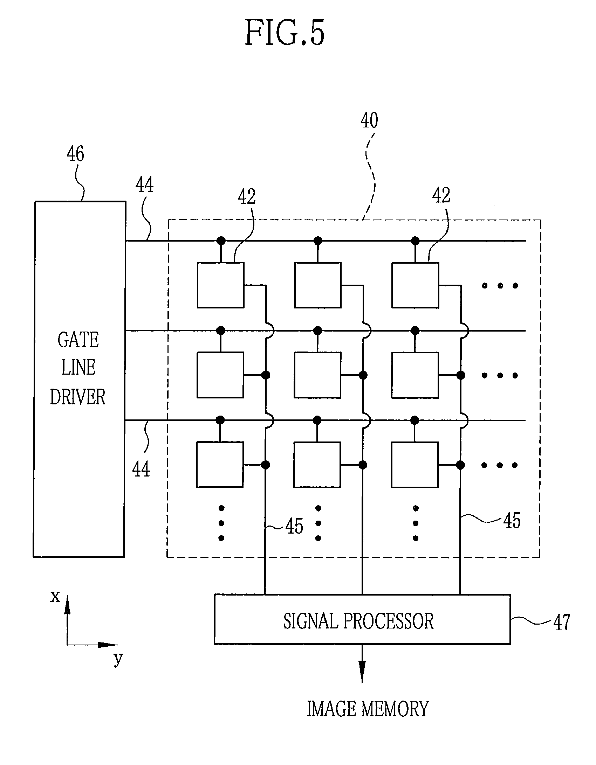

As shown in Fig. 5, on the TFT active matrix substrate 40, there are disposed in a matrix a plurality of pixel components 42 equipped with storage capacitors and TFTs. The storage capacitor accumulates the electric charges that have been generated in the charge generation layer. The TFTs read out the electric charges that have been accumulated in the storage capacitors. When the detection surface 24a is irradiated with X rays, the electric charges that have been generated in the charge generation layer of a particular pixel 42 is accumulated in the storage capacitor of the particular pixel 42. Thus, image information carried by the X rays irradiated to the detection surface 24a is converted into the electric charge information, and held in the cassette main body 24.

On the TFT active matrix substrate 40, there are disposed a plurality of gate lines 44, which extend in a constant direction (row direction) and are for switching ON and OFF the TFTs of the individual pixel components 42, and a plurality of data lines 45, which extend in a direction (column direction) orthogonal to the gate lines 44 and are for reading out the electric charges accumulated in the storage capacitors via the TFTs that have been switched ON. The individual gate lines 44 are connected to a gate line driver 46, and the individual data lines 45 are connected to a signal processor 47. When the electric charges are accumulated in the storage capacitors of the individual pixel components 42, the TFTs of the individual pixel components 42 are switched ON in order in row units by signals that are supplied via the gate lines 44 from the gate line driver 46, and the electric charges accumulated in the storage capacitors of the pixel components 42 whose TFTs have been switched ON are transmitted as electric charge signals through the data lines 45 and are inputted to the signal processor 47.

The signal processor 47 is equipped with amplifiers and sample/hold circuits that are disposed for each of the individual data lines 45, and the electric charge signals that have been transmitted through the individual data lines 45 are amplified by the amplifiers and thereafter are held in the sample/hold circuits. Further, multiplexers and A/D converters are connected in order to output sides of the sample/hold circuits. The electric charge signals held in the individual sample/hold circuits are inputted serially to the multiplexers, and are converted into digital image data by the A/D converters. The image data that has been outputted from the A/D converters of the signal processor 47 is stored in order in an image memory 49 (see Fig. 4). The image memory 49 has a storage capacity that is capable of storing X-ray image of at least one frame, however preferably, the image memory 49 has a storage capacity that is capable of storing X-ray image of plural frames.

As shown in Fig. 4, the cassette main body 24 includes a mounting detector 51, a first power source 52, a first controller 53, a notification section 54, and an imaging condition receiver 55. The mounting detector 51 detects whether or not the handle 23 is mounted to the cassette main body 24 by detecting output from signal lines of the connector 30 through the socket 29. Note that the mounting detector 51 may be configured so as to detect whether or not the handle 23 is mounted to the cassette main body 24 by utilizing light such as infrared rays, or an electrostatic effect (e. g. , a method of detecting, by the electrostatic capacity, whether or not an inductor is near). Alternatively, the mounting detector 51 may be configured so as to detect whether or not the connector 30 is inside the socket 29 by utilizing sensors or switches.

The first power source 52 includes a battery 52a and a power circuit. The power circuit supplies electric power from the battery 52a to the various circuits and elements in the cassette main body 24 such that image-taking can be performed in a state that the cassette main body 24 is removed from the handle 23. Further, the power circuit also has a function of charging the battery 52a when the cassette main body 24 is mounted to the handle 23. The battery 52a has a power capacity enough to take an image of at least one frame, however preferably, the battery 52a has a power capacity enough to take an image of plural frames.

The battery 52a may be a secondary battery such as nickel-cadmium battery, nickel metal-hydride battery, lithium-ion battery, and a capacitor such as electric double layer capacitor and lithium-ion capacitor. As to requirements to be fulfilled by the battery of the electronic cassette 12, the size of the battery is required to be small, the capacity of the battery is required to be relatively large, and the charging time for the battery is required to be short. Since a lithium-ion secondary battery has advantages such as higher energy density, smaller size, and larger capacity, in comparison with other secondary batteries, the lithium-ion secondary battery is preferably used in view of requirements such as miniaturization and large capacity. Additionally, since the capacitor enables fast charging, the capacitor is preferably used in view of requirements such as shortening of charging time.

Further, by utilizing nanoparticles, there have been recently developed new batteries such as a lithium-ion secondary battery having fast charging properties equal to that of the capacitor, and a lithium-ion capacitor having both fast charging properties and high energy density. It is more preferable that the lithium-ion secondary battery and the lithium-ion capacitor described above are used as the battery. (see http://www.toshiba.co.jp/about/press/2005_03/pr_j2901.htm and http://www.jmenergy.co.jp/img/JMenergy2009.pdf).

The first controller 53 consists of a microcomputer that includes a CPU which governs control of the entire cassette main body 24, a ROM serving as a storage medium for storing programs for an image data generating processing to be described later, a RAM serving as a work area for temporarily storing data, and a memory serving for storing various types of information.

The notification section 54 consists of LED lamps, a speaker for outputting a buzzer sound, and the like. Plural LED lamps are provided, and at least one of the plural LED lamps which are selected in accordance with the kind of each error is caused to light up. Alternatively, the buzzer sound is outputted at the rhythms changeable in accordance with the kind of each error. Accordingly, an error such as decrease in battery capacity or the like is notified, for example.

The imaging condition receiver 55 receives the imaging conditions such as irradiation time and the irradiation timing of the X rays from the console 13 in the state that the cassette main body 24 is removed from the handle 23. The first controller 53 controls the respective components based on the content received by the imaging condition receiver 55. Note that, the imaging condition receiver 55 receives the imaging conditions and the irradiation timing wirelessly or utilizing infrared rays, for example.

The handle 23 includes a communication section 57 for establishing communication with the console 13, a second power source 58, and a second controller 59. The communication section 57 has the socket 25 for the connection with the connector 17a of the cable 17, and a communication circuit (not shown). Communication between the communication section 57 and the console 13 is established while the cable 17 is connected to the socket 25.

The second power source 58 supplies electric power from the console 13 through the cable 17 to the various circuits and elements in the handle 23. Further, the second power source 58 supplies electric power to the first power source 52 through the connector 30 and the socket 29, while the cassette main body 24 is mounted to the handle 23. The first power source 52 supplies electric power from the second power source 58 to the various circuits and elements in the cassette main body 24, and charges the battery 52a.

The second controller 59 consists of a microcomputer that includes a CPU, a ROM, a RAM, and a memory, as in the case of the first controller 53. The second controller 59 controls the communication section 57 to establish communication between the first controller 53 and the console 13, while the cassette main body 24 is mounted to the handle 23.

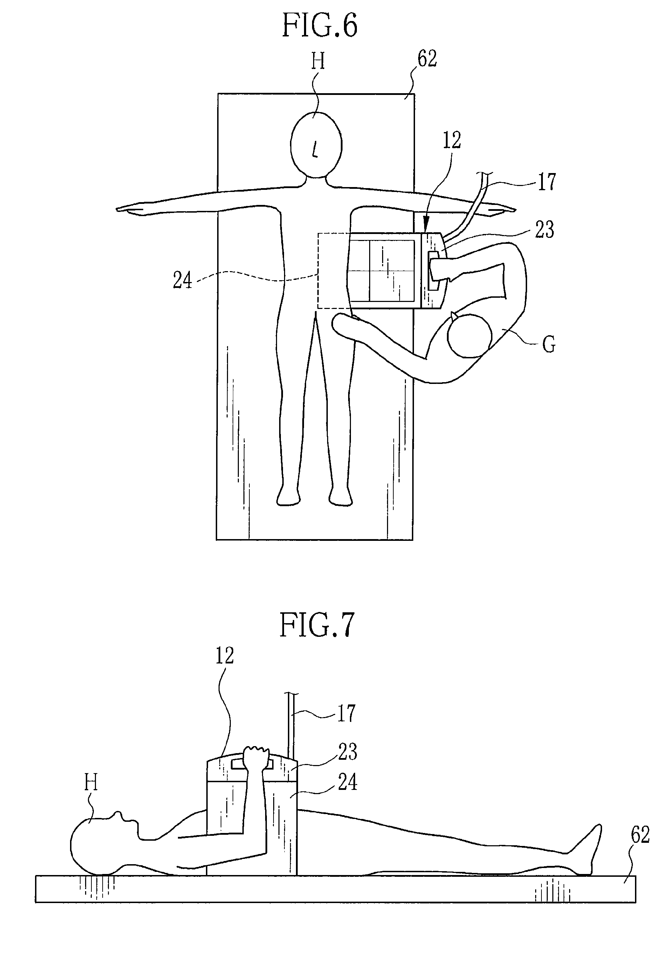

Next, an operation of the above embodiment is described. As shown in Fig. 2, the cassette main body 24 is mounted to the handle 23, and as a result, the electronic cassette 12 is suspended on the cable 17. For example, as shown in Fig. 6, when taking an image of the subject H lying on a bed 62, an operator G grips the handle 23 of the electronic cassette 12 and pulls the cable 17 so as to draw the cable 17 from the dolly section 20, and inserts the electronic cassette 12 under the subject H.

As the electronic cassette 12 includes the grid 35 made of lead, the TFT active matrix substrate 40 made of glass, the lead plate 37, and the like, the electronic cassette 12 is heavier than conventional film cassettes or CR cassettes. However, since the electronic cassette 12 is provided with the handle 23 and suspended on the cable 17, it is easy to handle the electronic cassette 12. Further, even if the operator G releases his/her hand from the electronic cassette 12 by mistake, as the spring balancer keeps the current length of the cable 17, it is possible to prevent the cable 17 from being drawn and the electronic cassette 12 from being dropped.

As shown in Fig. 7, for example, when taking an image of the subject H using the electronic cassette 12, the electronic cassette 12 is held upright by the subject H lying on the bed 62 in some cases. In this case, since the subject H can grip the handle 23 and the electronic cassette 12 is suspended on the cable 17, the posture of the electronic cassette 12 can be stably kept.

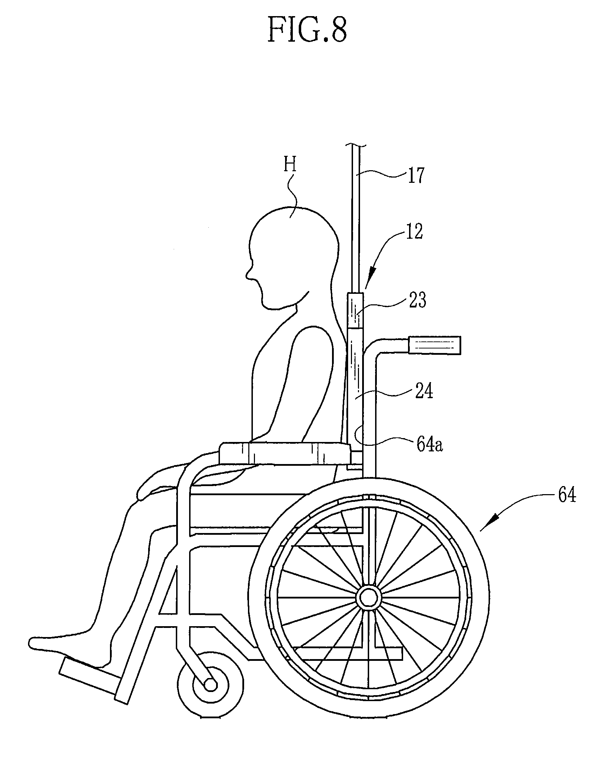

Further, as shown in Fig. 8, in the case where the electronic cassette 12 is inserted between the subject H sitting on a wheelchair 64 and a backrest 64a of the wheelchair 64 so as to take an image of the subject H, it is conventional that, since the electronic cassette 12 slides down by its weight to the seat of the wheelchair 64, it may be difficult to take an image of the subject H with the electronic cassette 12 kept at a predetermined position. However, according to this embodiment, since the electronic cassette 12 is suspended on the cable 17, even if the subject H does not have force enough to hold the electronic cassette 12 because of muscle weakness, the electronic cassette 12 can be kept at a predetermined position.

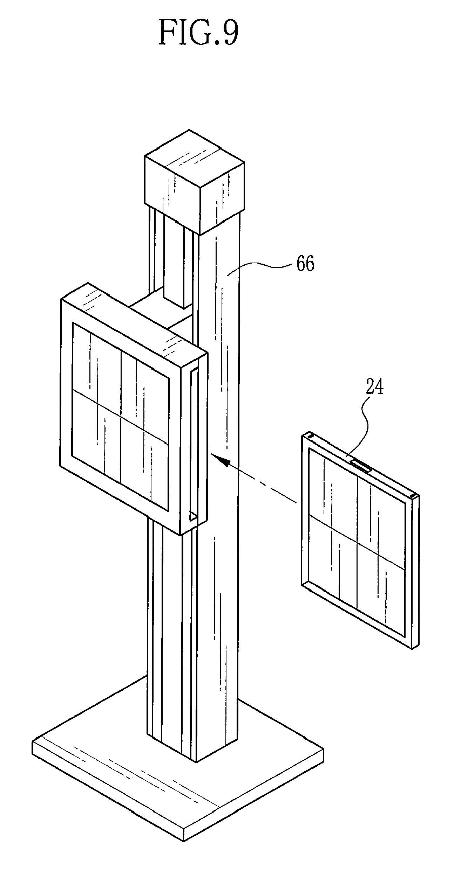

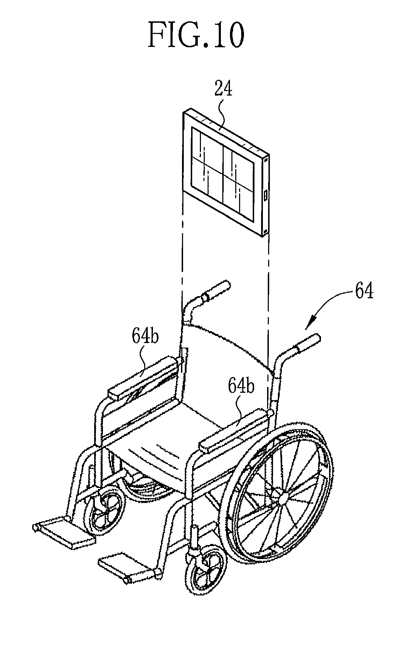

In the case where the cable 17 gets in the way of image-taking, image-taking can be performed in the state that the cassette main body 24 is removed from the handle 23. Thereby, as shown in Fig. 9, the cassette main body 24 can be set to a shooting table 66 for the CR cassette to perform image-taking, for example. Further, as shown in Fig. 10, the cassette main body 24 can be inserted between arm rests 64b of the wheelchair 64 such that long sides of the cassette main body 24 are aligned with a clearance between the arm rests 64b, so as to perform image-taking.

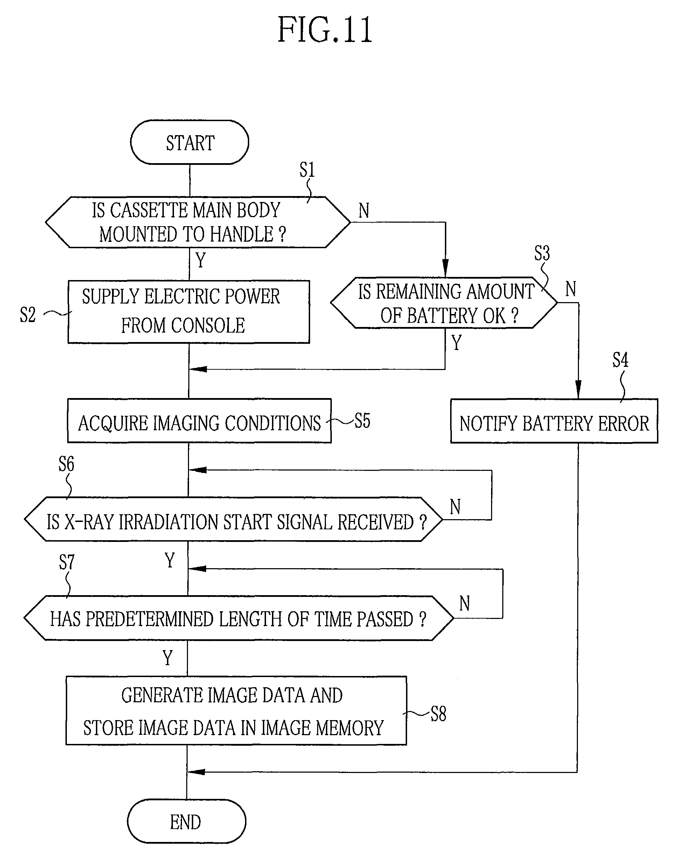

Next, by referring to a flowchart shown in Fig. 11, the image data generating processing using the electronic cassette 12 is explained. Firstly, the first controller 53 causes the mounting detector 51 to detect whether or not the cassette main body 24 is mounted to the handle 23 (S1).

In the case where the cassette main body 24 is mounted to the handle 23, the second controller 59 causes the second power source 58 to supply electric power from the console 13 through the cable 17 to the first power source 52 (S2). In the case where the cassette main body 24 is not mounted to the handle 23, the first controller 53 causes the first power source 52 to supply electric power to confirm remaining capacity of the battery 52a (S3). In the case where the remaining capacity of the battery 52a is less than the necessary capacity for taking an X-ray image of one frame, the first controller 53 causes the notification section 54 to notify that effect (S4).

The electronic cassette 12 establishes communication with the console 13 to acquire the imaging conditions such as the irradiation time of the X rays (S5). In the case where the cassette main body 24 is mounted to the handle 23, the second controller 59 controls the communication section 57 to establish communication with the console 13 through the cable 17. Further, in the case where the cassette main body 24 is not mounted to the handle 23, the first controller 53 controls the imaging condition receiver 55 to acquire the imaging conditions from the console 13.

Next, the electronic cassette 12 waits until an X-ray irradiation start signal representing the irradiation start of the X rays is inputted from the console 13, and causes the X-ray detection panel 36 to start image-taking (S6). At this time, as in the case of the time of acquiring the imaging conditions, the communication section 57 and the imaging condition receiver 55 are selectively utilized in accordance with whether or not the cassette main body 24 is mounted to the handle 23.

The first controller 53 refers to the imaging conditions, and judges whether or not a predetermined length of X-ray irradiation time has passed (S7). After the predetermined length of X-ray irradiation time has passed, the first controller 53 controls the gate line driver 46. The gate line driver 46 causes the signal processor 47 to read out the electric charges accumulated due to the irradiation of the X rays from the TFT active matrix substrate 40, and generates the image data of the X-ray image. The generated image data is stored in the image memory 49 (S8).

In the case where the cassette main body 24 is mounted to the handle 23, the second controller 59 reads out the image data from the image memory 49, and the communication section 57 sends the image data to the console 13. The console 13 subjects the image data sent thereto to various kinds of processing, and displays the images represented by the image data on the monitor 14.

In the case where the cassette main body 24 is not mounted to the handle 23, the image data remains to be stored in the image memory 49. When the cassette main body 24 is mounted to the handle 23, the second controller 59 reads out the image data from the image memory 49, and the communication section 57 sends the image data to the console 13. Accordingly, since the transmission of the image data which requires a large amount of electric power is performed after the cassette main body 24 is mounted to the handle 23, it is possible to prevent consumption of the battery 52a of the first power source 52.

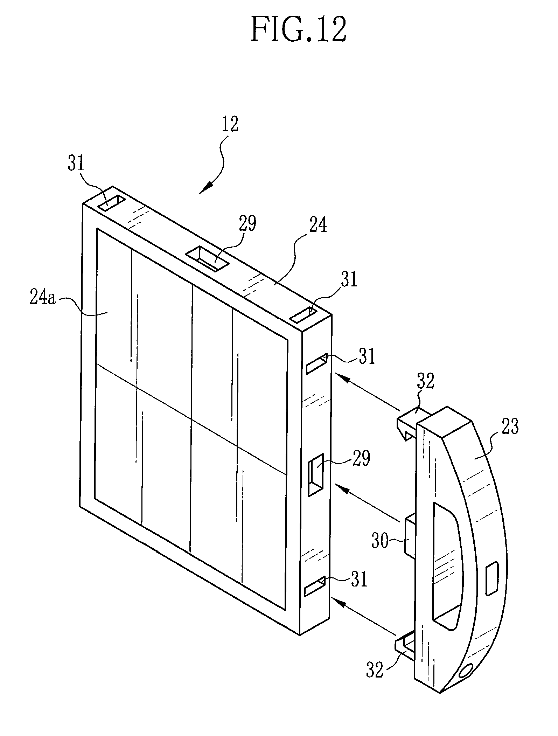

Although the second controller 59 is provided to the handle 23 in the above embodiment, only the first controller 53 may be provided to the handle 23 to take on the role of the second controller 59. Further, although the mounting direction of the cassette main body 24 to the handle 23 is limited to one direction in the above embodiment, the socket 29 and the pair of engagement holes 31 for mounting the cassette main body 24 to the handle 23 may be also provided in the longitudinal side surface of the cassette main body 24, as shown in Fig. 12. Thereby, it is possible to take an image of the subject H, while preventing the handle 23 from getting in the way of image-taking depending on the imaging procedure. Alternatively, a rail or the like to which the handle 23 can be mounted may be provided in the side surface of the cassette main body 24 such the handle 23 can be slid on the rail.

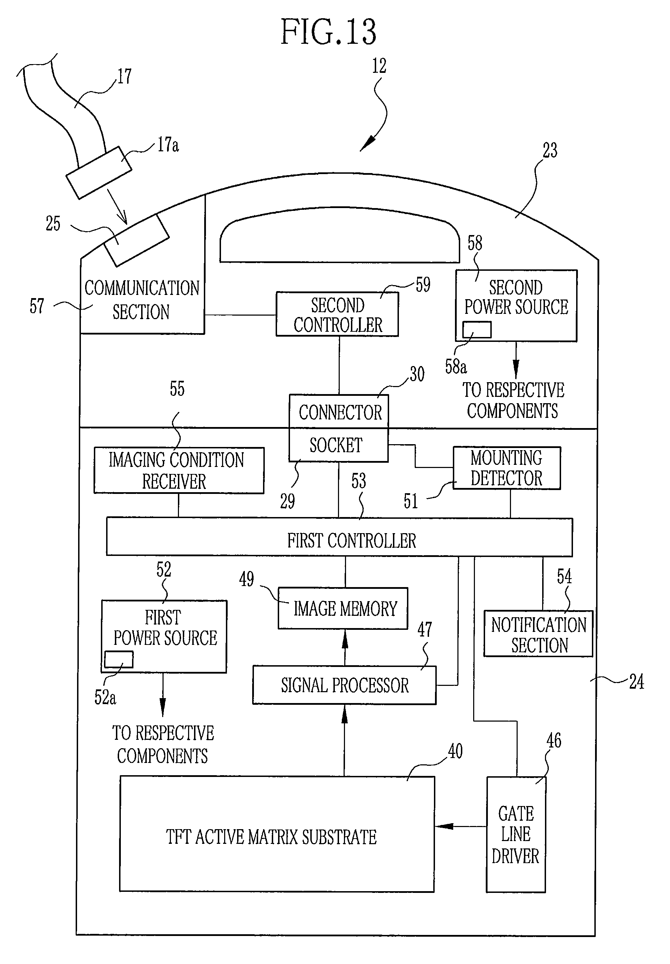

Although the electric power is supplied to the cassette main body 24 from the battery 52a of the first power source 52 when the cassette main body 24 is not connected to the cable 17 in the above embodiment, the present invention is not limited thereto. As shown in Fig. 13, the second power source 58 may be provided with a battery 58a for supplying electric power to the cassette main body 24. It is preferable that, the battery 58a of the second power source 58 is charged in the state that the cable 17 is connected to the handle 23, and the battery 58a of the second power source 58 supplies electric power to the respective components of the cassette main body 24 in the state that the cable 17 is removed from the handle 23. Thereby, even when the battery 52a of the first power source 52 runs out, the battery 58a of the second power source 58 can be used to supply electric power. Therefore, it is possible to increase the operation time of the electronic cassette 12 in the state that the cable 17 is not connected to the handle 23. As the battery 58a of the second power source 58, there may be used a variety of secondary batteries, capacitors, and the like, as in the case of the first power source 52.

Further, one handle 23 corresponds to one cassette main body 24 in the above embodiment, a plurality of the cassette main bodies 24 may be selectively mounted to one handle 23, for example. In this case, preferably, each of the cassette main bodies 24 is provided with an individual ID number, and the ID number is obtained by the console 13 through the handle 23 so as to be used for the control of each of the cassette main bodies 24. Note that, the sizes of the plurality of cassette main bodies 24 to be mounted to the handle 23 may be equal to or different from one another.

Although the X-ray detection panel 36 for converting X rays into digital data is a direct-conversion X-ray detection panel in which the TFT active matrix substrate 40 and the charge generation layer for absorbing the X rays and converting them into the electric charges are laminated in the above embodiment, a so-called indirect-conversion X-ray detection panel may be used. The indirect-conversion X-ray detection panel consists of a scintillator and a photoelectric conversion panel laminated on the scintillator. The scintillator converts X rays applied thereto into light. The photoelectric conversion panel detects the light converted by the scintillator. The scintillator may be, for example, gadolinium oxysulfide (GOS), cesium iodide (CsI), or the like.

Various changes and modifications are possible in the present invention and may be understood to be within the present invention.

## Claims

1. A radiographic image detection device (12) **characterized by** comprising:
a handle (23) suspended on a cable (17); and
a main body having a detector (24a) for detecting radioactive rays which has been transmitted through a subject (H) to generate a radiographic image, said main body (24) being suspended together with said handle (23) on said cable (17) when being mounted to said handle (23).

2. A radiographic image detection device (12) as defined in claim 1, **characterized in that** said main body (24) has a first power source (52) for supplying electric power when being removed from said handle (23), and a memory (49) for storing said radiographic image, such that image-taking is performed in a state that said main body (24) is removed from said handle (23).

3. A radiographic image detection device (12) as defined in claim 2, **characterized in that** said handle (23) has a communication section (57) for establishing communication with an external device through said cable (17).

4. A radiographic image detection device (12) as defined in claim 3, **characterized in that** said handle (23) has a second power source (58) for supplying electric power to said main body (24) when main body (24) is mounted to said handle (23).

5. A radiographic image detection device (12) as defined in claim 4, **characterized in that** said second power source (58) has a battery (58a) which is charged when said handle (23) is connected to said cable (17), and supplies electric power to said main body (24) when said handle (23) is removed from said cable (17).

6. A radiographic imaging system (10) **characterized by** comprising:
A. a radiation generator (11) for irradiating a subject (H) with radioactive rays;
B. a radiographic image detection device (12) including:
a handle (23) suspended on a cable (17); and
a main body (24) having a detector (24a) for detecting radioactive rays which has been transmitted through said subject (H) to generate a radiographic image, said main body (24) being suspended together with said handle (23) on said cable (17) when being mounted to said handle (23); and
C. a controller (13) for controlling said radiation generator (11) and said radiographic image detection device (12).
